# EUROPÄISCHE PATENTSCHRIFT

(11) **EP 3 791 912 B1**
(45) Veröffentlichungstag und Bekanntmachung des Hinweises auf die Patenterteilung: **06.09.2023**
(21) Anmeldenummer: 20000356.4
(22) Anmeldetag: 15.06.2018
(51) Int. Cl.: A61M 16/06

(54) **ATEMMASKE MIT KOPPELELEMENT FÜR EINE BÄNDERUNG**
RESPIRATORY MASK WITH A COUPLING ELEMENT FOR A HARNESS
MASQUE RESPIRATOIRE POURVU D'ÉLÉMENT DE COUPLAGE POUR UN AGENCEMENT DE BRIDE

(30) Priorität: 19.06.2017 DE 102017005691; 19.06.2017 DE 102017005704
(43) Veröffentlichungstag der Anmeldung: 17.03.2021
(62) Teilanmeldung aus: 18000527.4
(73) Patentinhaber: Löwenstein Medical Technology S.A., 2557 Luxembourg (LU)
(72) Erfinder: FRERICHS, Arnold, 21614 Buxtehude (DE); BECHTEL, Martin, 21423 Winsen / Luhe (DE); EIFLER, Martin, 25348 Glückstadt (DE); GARDEIN, Joachim, 38430 Icod de los Vinos Tenerife Islas Canarias (ES)
(74) Vertreter: Marx, Thomas

(56) Entgegenhaltungen:
- EP-A1- 2 818 196
- EP-A1- 2 954 920
- EP-A2- 1 057 494
- EP-A2- 2 732 839
- WO-A1-00/78384
- US-A1- 2004 182 398
- US-A1- 2005 011 521
- US-A1- 2011 048 425

## Beschreibung

Die Erfindung betrifft ein Patienteninterface mit einem Kopplungselement für eine Bänderung. Für die Befestigung eines Patienteninterface (PI) an einem Patienten oder Benutzer wird meist eine Bänderung verwendet. Das PI weist daher Kopplungselemente für die Bänderung auf. Bei einem PI welches als Beatmungsmaske mit einer Stirnabstützung ausgebildet ist, befinden sich üblicherweise zwei Kopplungselemente an der Stirnabstützung und zwei Kopplungselemente an dem Maskenkörper. Die Verbindung von Kopplungselement und Bänderung kann unter Vermittlung von zusätzlichen Klips oder Adaptern erfolgen. Alternativ kann die Bänderung Schlaufen ausbilden, die unmittelbar an dem Kopplungselement befestigt werden. Die unterschiedlichen Verbindungen von Kopplungselement und Bänderung sind beispielsweise in der EP 2727620 A1 oder der US 20040045551 A1 beschrieben. Die bekannten Lösungen wiesen für ein gegebenes Bänderungsende immer nur ein definiertes Kopplungselement auf. Durch diese feste Zuordnung ist eine individuelle Anpassung für den Patienten meist nicht möglich.

Die EP 2 818 196 A1 offenbart ein Patienteninterface mit einer Stirnstütze und einem Kopplungselement für eine Bänderung und vier Aufnahmemittel, die unterschiedliche Möglichkeiten der Ankopplung einer Bänderung bieten und somit zwei Ebenen bilden. Die EP 2 818 196 A1 weist einen federnden Verstellmechanismus auf, daher ist die Stirnstütze nicht einstückig aufgebaut. Zudem weist die Stirnstütze eine sehr geringe Neigung zu der Stirn eines Nutzers auf, weshalb eine Ebene, die an der Basis der Stirnstütze liegt, einen geringen Abstand zu den Ebenen der Aufnahmemittel aufweist. Diese aus vielen Teilen bestehende Stirnstütze ist nur aufwändig herstellbar, teuer und nicht leicht zu reinigen.

Die EP 2 954 920 A1 offenbart ein Patienteninterface mit einer einstückigen Stirnstütze und einem Kopplungselement für eine Bänderung und Aufnahmemittel die eine Ebene bilden. Da die Stirnstütze keine Verstellfunktion aufweist ist die Neigung der Stirnstütze zu der Stirn eines Nutzers relativ groß. Eine Ebene, die an der Basis der Stirnstütze liegt, weist daher einen großen Abstand zu der Ebenen des Aufnahmemittels auf. Die Lösung der EP 2 954 920 A1 bietet eine nicht ausreichende individuelle Anpassung der Stirnstütze für den Patienten.

Es ist daher die Aufgabe der vorliegenden Erfindung ein Patienteninterface mit einem Kopplungselement für eine Bänderung anzugeben, dass eine individuelle Anpassung der Stirnstütze für den Patienten ermöglicht und zugleich für eine Vielzahl von Patienten anwendbar ist, ohne ein Stirnpolster und/oder einen Verstellmechanismus zu benötigen und trotzdem aus wenigen Teilen besteht, leicht und kostengünstig herstellbar ist und leicht zu reinigen ist.

Die Aufgabe wird gelöst durch unabhängigen Anspruch 1. Bevorzugte Ausführungsformen" sind in den abhängigen Ansprüchen definiert.

Das Patienteninterface ist alternativ auch dadurch gekennzeichnet, dass der gebogene Konturverlauf einen ansteigenden Krümmungsradius aufweist.

Das Patienteninterface ist alternativ auch dadurch gekennzeichnet, dass der gebogene Konturverlauf von Aufnahmemittel 6 von einem mittleren Bereich des Ausnahmemittels ausgehend einen ansteigenden Krümmungsradius aufweist.

Das Patienteninterface ist alternativ auch dadurch gekennzeichnet, dass die die Aufnahmemittel 6 einen gebogenen Konturverlauf aufweisen, wobei die Aufnahmemittel 6 zumindest abschnittsweise konvex oder konkav verlaufen.

Das Patienteninterface ist alternativ auch dadurch gekennzeichnet, dass die die Aufnahmemittel 5 und/oder 6 einen gebogenen Konturverlauf aufweisen, der zumindest abschnittsweise von der Stirn eines Benutzers weg weist.

Das Patienteninterface ist alternativ auch dadurch gekennzeichnet, dass die die Aufnahmemittel 5 und/oder 6 einen gebogenen Konturverlauf aufweisen, sodass die die Aufnahmemittel 5 und/oder 6 nur abschnittsweise, beispielsweise im mittleren Abschnitt, an der Stirn eines Benutzers anliegen.

Das Patienteninterface ist ergänzend auch dadurch gekennzeichnet, dass die beiden Aufnahmemittel geometrisch unterschiedlich ausgebildet sind.

Das Patienteninterface ist alternativ oder ergänzend auch dadurch gekennzeichnet, dass die Aufnahmemittel als geschlossener oder geschlitzter Steg ausgebildet sind.

Das Patienteninterface ist ergänzend auch dadurch gekennzeichnet, dass das Kopplungselement einstückig als Teil einer Stirnabstützung eines Patienteninterface ausgebildet ist und die Symmetrieebene S mit der Längsachse der Stirnabstützung zusammenfällt.

Das Patienteninterface ist alternativ oder ergänzend auch dadurch gekennzeichnet, dass die geschlitzten Stegen 6 außen und die geschlossenen Stege 5 relativ zu den geschlitzten Stegen weiter innen, näher an der Symmetrieebene S angeordnet sind.

Das Patienteninterface ist alternativ oder ergänzend auch dadurch gekennzeichnet, dass die geschlitzten Stegen und die geschlossenen Stege relativ zu der Symmetrieebene S unsymmetrisch angeordnet sind.

Das Patienteninterface ist alternativ oder ergänzend auch dadurch gekennzeichnet, dass die geschlitzten Stege und die geschlossenen Stege zumindest abschnittsweise parallel zueinander verlaufen. Das Patienteninterface ist alternativ oder ergänzend auch dadurch gekennzeichnet, dass die geschlitzten Stege und/oder die geschlossenen Stege zumindest abschnittsweise windschief zueinander und/oder zur Symmetrieebene S verlaufen.

Das Patienteninterface ist alternativ oder ergänzend auch dadurch gekennzeichnet, dass die Aufnahmemittel unterschiedlich weit von der Ebene E entfernt angeordnet sind.

Das Patienteninterface ist alternativ oder ergänzend auch dadurch gekennzeichnet, dass die Stege im Querschnitt gerundet oder eckig sind.

Das Patienteninterface ist alternativ oder ergänzend auch dadurch gekennzeichnet, dass die Stege zumindest abschnittsweise eine raue Oberfläche aufweisen.

Das Patienteninterface ist alternativ oder ergänzend auch dadurch gekennzeichnet, dass die geschlitzten Stege andere Oberflächen aufweisen, als die geschlossenen Stege.

Das Patienteninterface ist alternativ oder ergänzend auch dadurch gekennzeichnet, dass die Stege Haltemittel für die Bänderung aufweisen.

Das Patienteninterface ist alternativ oder ergänzend auch dadurch gekennzeichnet, dass ein geschlitzter Steg zwischen den beiden Teilstegen jeweils eine Öffnung für die Bänderung bereitstellt und Öffnung so dimensioniert ist, dass die Bänderung mit ihrer flachen Seite hindurchgefädelt werden kann.

Das Patienteninterface ist alternativ oder ergänzend auch dadurch gekennzeichnet, dass die beiden Teilstege mit einem Versatz zueinander angeordnet sind.

Das Patienteninterface ist alternativ oder ergänzend auch dadurch gekennzeichnet, dass die Teilstege unterschiedlich lang sind.

Das Patienteninterface ist alternativ oder ergänzend auch dadurch gekennzeichnet, dass die Teilstege benachbart zur Öffnung Sicherungselemente aufweisen, die ein hinausrutschen der Bänderungsschlaufe aus der Öffnung verhindern.

Die Begriffe Patienteninterface und Atemmaske werden synonym verwendet. Im Sinne der Erfindung ist jedes Interface für die Beatmung umfasst.

So kann das Kopplungselement 1, zum Beispiel in Form einer Lasche, aus demselben biegsamen Material wie das PI hergestellt sein. Infolgedessen ist es zum Beispiel bevorzugt, wenn das Kopplungselement mit dem PI einstückig ausgebildet ist oder zweistückig mit dem Grundkörper des PI über eine Schnittstelle gekoppelt ist. Das Kopplungselement und das PI sind bevorzugt aus Polycarbonat, Polyethylen oder ähnlichen Kunststoffen im Spritzgussverfahren hergestellt.

Das Kopplungselement kann ein Polster aufweisen, welches der Abstützung an der Haut eines Benutzers dient.

Das Kopplungselement kann sich auch unmittelbar an der Haut eines Benutzers abstützen.

Das Kopplungselement kann auch so gestaltet sein, dass ein Aufnahmemittel 5, 6 mit einem Bänderungsende 4 zumindest teilweise bedeckt ist und daher die Bänderung an der Haut eines Benutzers anliegt.

Das Kopplungselement kann einstückig als Teil einer Stirnabstützung ausgebildet sein.

Das Kopplungselement 1 weist zumindest eine Symmetrieebene S auf. Das Kopplungselement 1 weist senkrecht zu der Symmetrieebene S eine gedachte Ebene E auf, die parallel zu der Anlagefläche (beispielsweise Stirn) eines Benutzers verläuft. Das Kopplungselement 1 erstreckt sich zumindest abschnittsweise abgewinkelt von der Symmetrieebene entlang der Ebene E mit einer gerundeten Kontur. Von der Symmetrieeben beabstandet befinden sich zumindest vier Aufnahmemittel 5, 6 für ein Bänderungsende 4 einer Bänderung 3. Die Aufnahmemittel 5, 6 sind in Paaren beidseitig der Symmetrieebene S angeordnet. Das Aufnahmemittel 5 ist räumlich benachbart zum Aufnahmemittel 6 und näher zu der Symmetrieebene S angeordnet als das Aufnahmemittel 6. Durch den gerundeten Konturverlauf des Kopplungselementes 1 befinden sich die Aufnahmemittel 5, 6 unterschiedlich weit von der Ebene E entfernt. Das Aufnahmemittel 6 ist näher an der Ebene E angeordnet als das Aufnahmemittel 5.

Ein Bänderungsende 4 kann alternativ an dem Aufnahmemittel 5 oder an dem Aufnahmemittel 6 auf einer Seite des Kopplungselementes befestigt werden. Da das Aufnahmemittel 5 näher zu der Symmetrieebene S angeordnet ist als das Aufnahmemittel 6, kann dadurch eine Feineinstellung der Länge des Bänderungsendes 4 erfolgen. Die Aufnahmemittel 5, 6 befinden sich zudem unterschiedlich weit von der Ebene E entfernt. Dadurch kann eine Feineinstellung des Verlaufes der Bänderung erfolgen.

Das Kopplungselement kann auch einstückig als Teil eines Grundkörpers eines PI ausgebildet sein.

Das Kopplungselement weist zumindest vier Aufnahmemittel 2 für Bänderungsenden 4 einer Bänderung 3 auf. Die Aufnahmemittel 2 sind als geschlossener 5 oder geschlitzter Steg 6 ausgebildet. Die Bänderungsenden 4 werden zur Verbindung mit den Stegen 5, 6 mit sich selbst geschlauft 7 und durch einen Klett 8 fixiert. Die Schlaufen 7 umfassen den geschlossenen oder den geschlitzten Steg zumindest teilweise.

Das Kopplungselement 1 weist eine Symmetrieebene S auf von der die zumindest vier Aufnahmemittel 2 beabstandet sind.

Die Aufnahmemittel 2 können so am Kopplungselement 1 angeordnet sein, dass die geschlossenen Stege 5 außen und die geschlitzten Stegen 6 relativ zu den geschlossenen Stegen weiter innen, näher an der Symmetrieebene S angeordnet sind.

Die Aufnahmemittel 2 können so am Kopplungselement 1 angeordnet sein, dass die geschlitzten Stegen 6 außen und die geschlossenen Stege 5 relativ zu den geschlitzten Stegen weiter innen, näher an der Symmetrieebene S angeordnet sind.

Die Aufnahmemittel 2 können so am Kopplungselement 1 angeordnet sein, dass die geschlitzten Stegen 6 und die geschlossenen Stege 5 relativ zu der Symmetrieebene S unsymmetrisch angeordnet sind.

Die Aufnahmemittel 2 können so am Kopplungselement 1 angeordnet sein, dass die geschlitzten Stegen 6 und die geschlossenen Stege 5 relativ zu der Symmetrieebene S symmetrisch angeordnet sind.

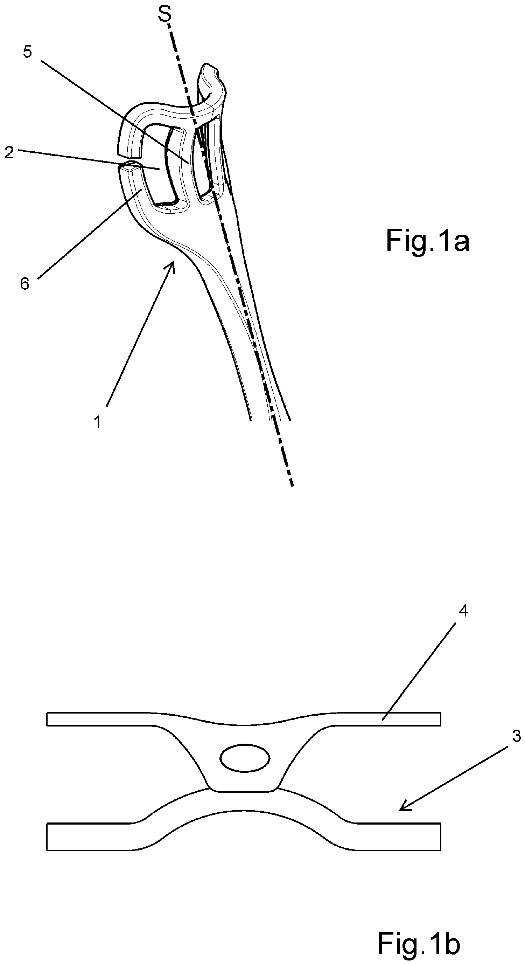

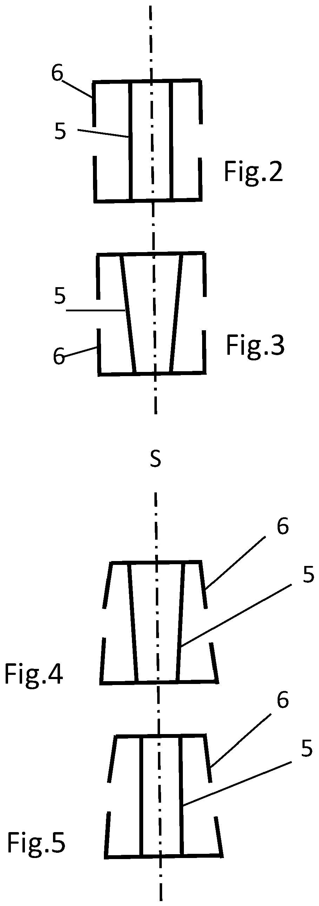

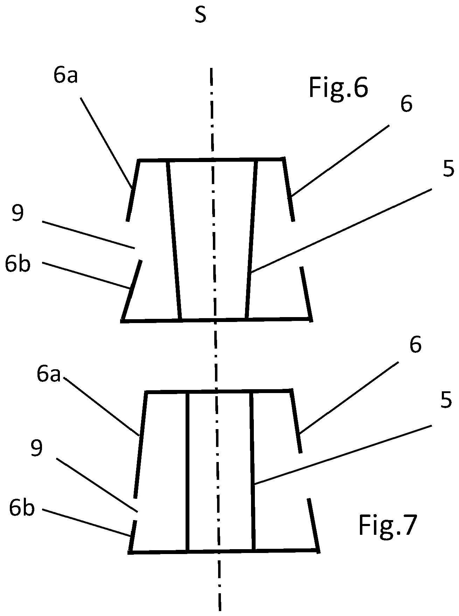

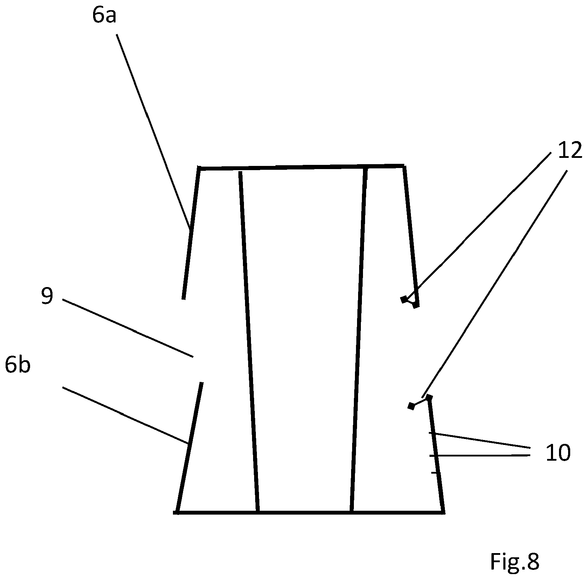

Fig. 2 Die Aufnahmemittel 2 können so am Kopplungselement 1 angeordnet sein, dass die geschlitzten Stege 6 und die geschlossenen Stege 5 zumindest abschnittsweise parallel zueinander verlaufen.

Die Aufnahmemittel 2 können so am Kopplungselement 1 angeordnet sein, dass die geschlitzten Stege 6 und die geschlossenen Stege 5 zumindest abschnittsweise eine Parallelverschiebung aufweisen. Fig. 3 - 5 Die Aufnahmemittel 2 können so am Kopplungselement 1 angeordnet sein, dass die geschlitzten Stege 6 und/oder die geschlossenen Stege 5 zumindest abschnittsweise windschief verlaufen.

In Fig. 3 sind die die geschlitzten Stege 6 parallel zueinander und die geschlossenen Stege 5 windschief.

In Fig. 4 sind die die geschlitzten Stege 6 zueinander und zu den geschlossenen Stegen 5 windschief. Ebenso sind die geschlossenen Stegen 5 zueinander und zu den geschlitzten Stege 6 windschief

In Fig. 5 sind die geschlossenen Stege 5 zueinander parallel und die geschlitzten Stege 6 zueinander windschief.

Die Stege 5,6 können im Querschnitt gerundet oder eckig sein. Die Stege können beispielsweise auch eine glatte Oberfläche aufweisen. Alternativ ist auch vorgesehen, dass die Stege 5,6 zumindest abschnittsweise eine raue Oberfläche aufweisen.

Die Stege 5,6 können auch Haltemittel 10 für die Bänderung aufweisen. Die Haltemittel sind beispielsweise Haken oder Dornen die sich nur wenig über die Oberfläche erheben.

Ein geschlitzter Steg 6 bietet zwischen den beiden Teilstegen 6a, 6b jeweils eine Öffnung 9 für die Bänderung. Die Öffnung ist bevorzugt so dimensioniert, dass die Bänderung mit ihrer flachen Seite hindurchgefädelt werden kann.

Die geschlitzten Stege 6 können andere Oberflächen aufweisen, als die geschlossenen Stege 5.
Fig. 6

Die geschlitzten Stege 6 können fluchtend oder mit einem Versatz zueinander angeordnet sein
Fig. 7

Die geschlitzten Stege 6 können gleichlange oder unterschiedlich lange Teilstege aufweisen.
Fig. 8

Die Teilstege können benachbart zur Öffnung 9 Sicherungselemente 12 aufweisen, die ein Hinausrutschen der Bänderungsschlaufe aus der Öffnung verhindern.

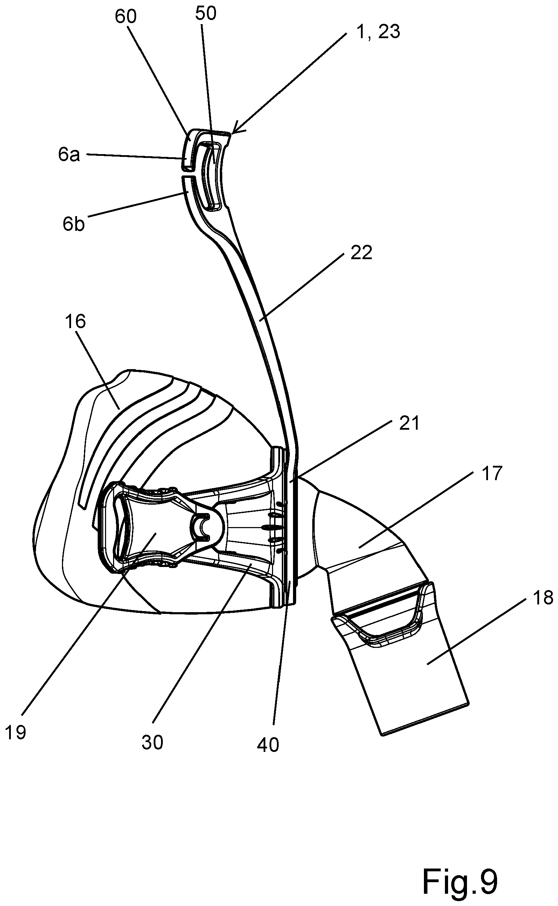

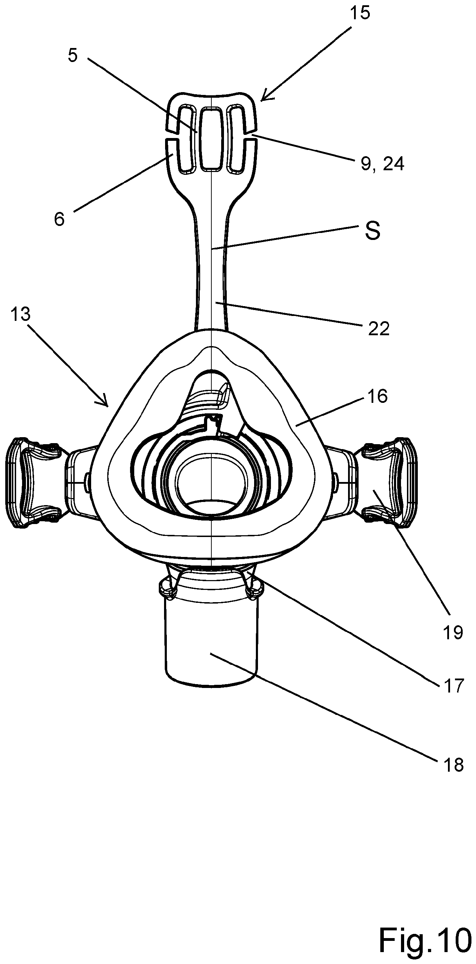

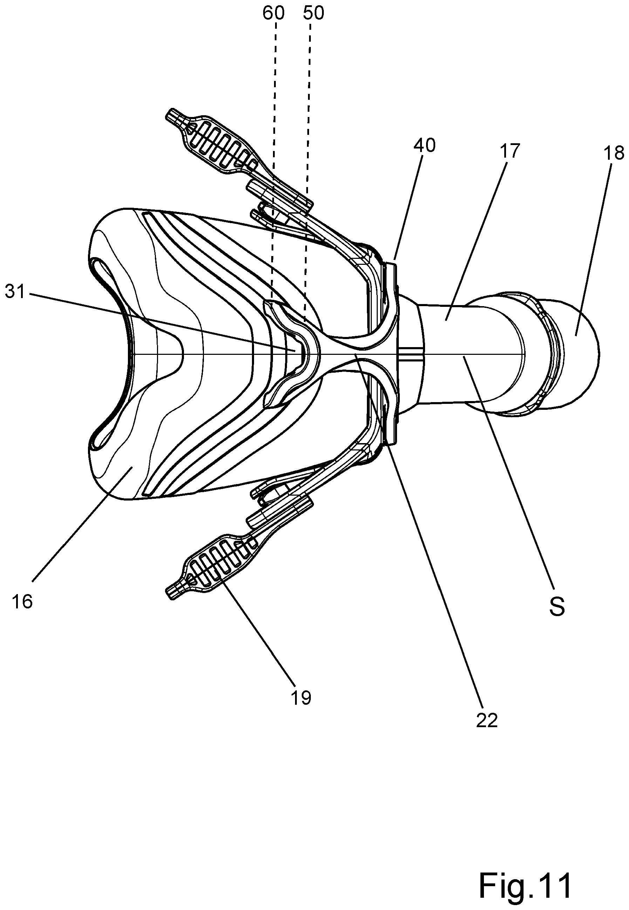

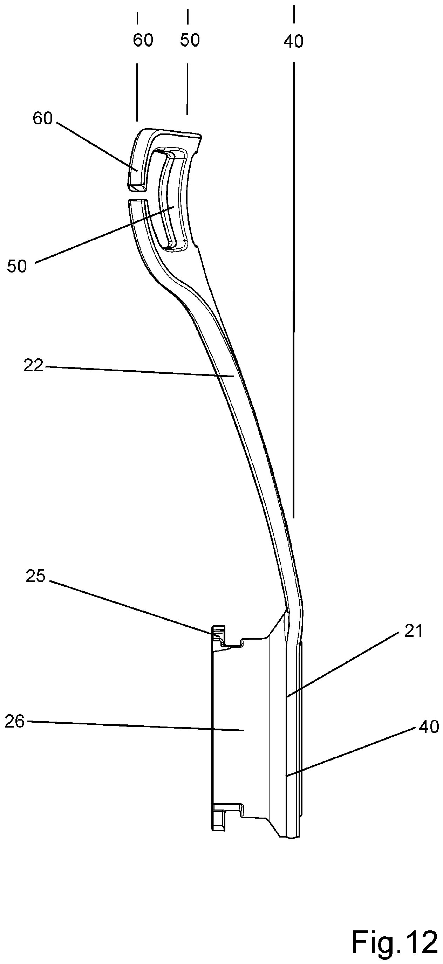

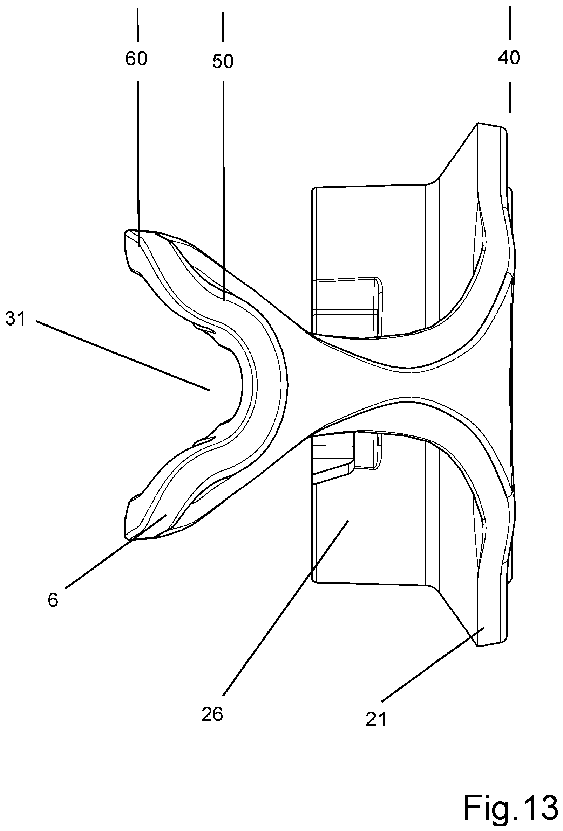

Die Fig. 9 bis 13 zeigen die erfindungsgemäße Atemmaske. Die erfindungsgemäße Atemmaske ist modular aufgebaut. Als Basis der Atemmaske (13) dient der Maskenkörper (14). Dieser ist beispielsweise als Spritzgussteil aus Kunststoff relativ fest ausgeführt. An dem Maskenkörper (14) setzen die Stirnstütze (15), die einen sicheren Sitz der Maske auf dem Gesicht eines nicht dargestellten Patienten gewährleistet, und der Maskenwulst (16), der durch seine Passform und seine elastische Beschaffenheit für einen dichten Abschluss der Atemmaske am Patienten sorgt, an. Das Anschlussstück (17) stellt die Verbindung zu einem nicht dargestellten Schlauch dar, über den das Atemgas von einem Beatmungsapparat zur Atemmaske transportiert wird. Der Schlauch wird mittels einer auf dem Anschlussstück (17) drehbeweglich gelagerten Hülse (18) mit dem Anschlussstück (17) verbunden. Mithilfe von seitlich mit dem Maskenkörper (13) verbindbaren Clips (19) kann die Atemmaske durch Bänder sicher am Kopf des Patienten fixiert werden.

Zu erkennen ist die Symmetrie des Aufbaus entlang der vertikalen Mittelachse (s). An beiden Seiten des Maskenkörpers (13) befindet sich hier ein Clip (19) zur Bandaufnahme, jedoch ist auch eine asymmetrische Ausführungsform denkbar. Dies kann beispielsweise umgesetzt werden, indem auf einer Seite die Verbindung von Atemmaske und Halteband mit einem Clip (19) realisiert wird, während auf der anderen Seite eine Schlaufe und ein Haken genutzt werden.

Zu sehen ist eine trianguläre Grundform des Maskenwulstes (16) sowie das Innere der Atemmaske durch die patientenseitige Öffnung des Maskenwulstes hindurch. An der oberen Seite des Maskenwulstes (3) ragt die Stirnstütze (15) hervor. An den Seiten der Atemmaske sind die mit dem Maskenkörper (13) verbundenen Clips (19) zur Bandankopplung zu sehen. An der unteren Seite des Maskenwulstes (16) ragt das Anschlussstück (17) mit der montierten Hülse (18) hervor.

In einer Ansicht ist eine der menschlichen Anatomie angepasste Formgebung der Stirnstütze (15) erkennbar. Aus der sich aus der Verbindung mit dem Maskenkörper (13) ergebenden Fläche ragt das Kopfende der Stirnstütze (15) in Richtung eines Patienten, um auch ohne übermäßiges Neigen der gesamten Atemmaske eine ausreichende Stützfunktion an der Stirn eines Patienten zu erreichen.

Die Basis der Stirnstütze (13) bildet der Stirnstützenverbinder (21), mit dem die Stirnstütze (13) am Maskenkörper befestigt wird. Ein Steg (22) verbindet den Stirnstützenverbinder (21) mit der Bandankopplung (1, 23) am Kopf der Stirnstütze. Die Bandankopplung (23) besteht aus drei horizontal nebeneinander angeordneten, in vertikaler Richtung länglich ausgedehnten Aussparungen in der Struktur der Stirnstütze. Die beiden äußeren Aussparungen weisen jeweils einen zusätzlichen Spalt (9,24) in der nach außen gewandten Struktur der Stirnstütze auf. Diese Spalte (24) können zum Einfädeln der Haltebänder auf Stirnhöhe eines Patienten in die Bandankopplung (23) genutzt werden. Alternativ kann das Band auch durch die mittig positionierte Aussparung geführt werden.

Es ist auch an andere Ausführungsformen der Bandankopplung (23) gedacht. Beispielsweise kann diese auch nur durch zwei Aussparungen mit zusätzlichem Spalt (24) umgesetzt werden.

Der Steg (22) ist in seiner Breite in der gezeigten Ausführungsform gegenüber dem Kopfteil mit Bandankopplung (23) und dem Stirnstützenverbinder (21) signifikant reduziert. Dies ermöglicht ein ansprechendes, schlankes Design und die Einsparung von Material.

Der Stirnstützenverbinder (21) bietet erfindungsgemäß zur Vervollständigung des im Maskenkörper (1) angelegten Bajonettverschlusses eine in Positionierung und Anzahl der im Maskenkörper vorhandenen Kanäle entsprechende Anzahl an Verschlusszähnen (25). Diese stehen auf der äußeren Kontur des Verbindungsringes (26) radial nach außen und weisen ihrerseits jeweils einen weiteren radial nach außen gerichteten Zahn auf, der gemeinsam mit dem Rastzahn im Maskenkörper für die Arretierung von Maskenkörper (30) und Stirnstütze (13) in der vorgesehenen Position sorgt. Die innere Kontur des Stirnstützenverbinders (21) ist erfindungsgemäß als Kugelkäfig (27) ausgeführt, der das Anschlussstück (17) beweglich lagern kann. Zum Anschlussstück (17) hin wird der Kugelkäfig (27) durch eine sich nach außen verengende Ringstruktur (28) begrenzt.

Zu erkennen ist, dass das Kopfteil der Stirnstütze (13) in Relation zum Stirnstützenverbinder (21) zu einem Patienten hin ragt. Die . Form des die Bandankopplung (23) beherbergenden Kopfes der Stirnstütze (2) weist eine Aussparung (31) auf, die zwischen der Stirn eines Patienten und der Bandankopplung (23) in der Stirnstütze (2) einen Raum bietet. Die Aussparung (31) weist gemeinsam mit den Strukturelementen der Bänderungsankopplung, in einer Ansicht von oben eine Y-Form auf. Der Konturverlauf im Bereich der Aussparung (31) verläuft gerundet mit einem oder mehreren Radien. Entsprechend des Materials der Bänderungsankopplung und der Wandstärken kann die Y-Struktur eine Elastizität bieten, die eine Adaptation des Kopfteils der Stirnstütze (13) relativ zur Stirn des Patienten erlaubt.

Bei einer rotationssymmetrischen Ausführung des Maskenkörpers (30) kann dieser in genau zwei um 180° verschobenen Positionen mit der Stirnstütze (13) verbunden werden, die funktional identisch sind. Damit lässt sich eine Vereinfachung der Montage der Module der Atemmaske erreichen.

Das Anschlussstück (17) ist als abgewinkelte Rohrstruktur ausgeführt und weist an einem Ende eine Oberflächenkontur (32) auf, die einer Teilkugel entspricht. Diese Teilkugel (32) dient der beweglichen Verbindung von Anschlussstück (17) und Stirnstütze (13) mittels des im Stirnstützenverbinder (21) realisierten Kugelkäfigs (27).

Am anderen Ende ist eine drehbar gelagerte Hülse (18) als Anschluss für einen nicht dargestellten Schlauch montiert.

Der Stirnstützenverbinder (21) definiert eine erste Ebene (40). Im Bereich der Bänderungsankopplung der Stirnstütze wird eine zweite Ebene (50) durch die Aufnahmemittel 5 gebildet. Im Bereich der Bänderungsankopplung der Stirnstütze wird auch eine dritte Ebene (60) durch die Aufnahmemittel 6 gebildet.

Die Ebenen sind im Wesentlichen parallel zueinander angeordnet. Dabei ist die erste Ebene (40) weiter von der dritten Ebene (60) beabstandet als von der zweiten Ebene (50) . Der Versatz von der ersten Ebene (40) zu der zweiten Ebene (50) beträgt 1 cm bis 4 cm, bevorzugt 1,2 cm bis 3 cm. Der Versatz von der zweiten Ebene (50) zu der dritten Ebene (60) beträgt 3 mm bis 23 mm, bevorzugt 4 mm bis 15 mm.

Die Ebenen sind alternativ in einem Winkel zueinander angeordnet. Dabei ist zweiten Ebene (50) in einem Winkel von 1° bis 20° zur ersten Ebene (40) in Richtung auf die Stirn eines Patienten geneigt. Alternativ oder ergänzend ist die dritte Ebene (60) in einem Winkel von 1° bis 20° zur ersten Ebene (40) in Richtung auf die Stirn eines Patienten geneigt. Alternativ oder ergänzend ist die dritte Ebene (60) in einem Winkel von 1° bis 20° zur zweiten Ebene (40) geneigt.

## Patentansprüche

1. Patienteninterface mit zumindest einem Kopplungselement (1) für eine Bänderung, wobei das Kopplungselement (1) zumindest eine Symmetrieebene S aufweist und zumindest vier Aufnahmemittel (5), (6) für ein Bänderungsende (4) einer Bänderung (3) wobei die Aufnahmemittel (5), (6) in Paaren beidseitig der Symmetrieebene (S) angeordnet sind und ein Bänderungsende (4) alternativ an dem Aufnahmemittel (5) oder an dem Aufnahmemittel (6) auf einer Seite des Kopplungselementes befestigbar ist und das Aufnahmemittel (5) räumlich benachbart zum Aufnahmemittel (6) und näher zu der Symmetrieebene (S) angeordnet ist als das Aufnahmemittel (6),
wobei das Kopplungselement (1) einstückig als Teil einer Stirnabstützung (15) Patienteninterface ausgebildet ist und die Symmetrieebene (S) mit der Längsachse der Stirnabstützung zusammenfällt, wobei das Kopplungselement (1) senkrecht zu der Symmetrieebene (S) eine gedachte Ebene (E) aufweist, die parallel zu der Stirn eines Benutzers verläuft, wobei durch die Aufnahmemittel (5) eine Ebene (50) gebildet ist und durch die Aufnahmemittel (6) eine weitere Ebene (60) gebildet wird, wobei die Ebenen (50, 60) voneinander beabstandet sind, wobei im Bereich des Patienteninterface eine erste Ebene (40) im Bereich der Basis der Stirnstütze definiert ist, im Bereich der Bänderungsankopplung der Stirnstütze durch die Aufnahmemittel (5) eine zweite Ebene (50) gebildet ist und im Bereich der Bänderungsankopplung der Stirnstütze durch die Aufnahmemittel (6) eine dritte Ebene (60) gebildet wird, wobei die erste Ebene (40) weiter von der dritten Ebene (60) beabstandet ist als von der zweiten Ebene (50), wobei die Ebenen im Wesentlichen parallel zueinander angeordnet sind und wobei der Versatz von der ersten Ebene (40) zu der zweiten Ebene (50) 1 cm bis 4 cm beträgt und der Versatz von der zweiten Ebene (50) zu der dritten Ebene (60) 3 mm bis 23 mm beträgt, wobei ein Stirnstützenverbinder (21) die Basis der Stirnstütze (15) bildet und mit dem Stirnstützenverbinder (21) die Stirnstütze am Maskenkörper (13) befestigt wird,
wobei ein Steg (22) den Stirnstützenverbinder (21) mit dem Kopplungselement (1) für eine Bänderung der Stirnstütze (15) verbindet und,
wobei der Stirnstützenverbinder (21) zur Vervollständigung eines im Maskenkörper (1) angelegten Bajonettverschlusses eine in Positionierung und Anzahl der im Maskenkörper vorhandenen Kanäle entsprechende Anzahl an Verschlusszähnen (25) bietet,
wobei diese Verschlusszähne (25) auf der äußeren Kontur eines Verbindungsringes (26) radial nach außen stehen und ihrerseits jeweils einen weiteren radial nach außen gerichteten Zahn aufweisen, der gemeinsam mit einem Rastzahn im Maskenkörper für die Arretierung von Maskenkörper und
Stirnstütze in der vorgesehenen Position sorgt,
wobei die innere Kontur des Stirnstützenverbinders (21) als ein Kugelkäfig (27) ausgeführt ist, der das Anschlussstück (17) beweglich lagert,
wobei der Kugelkäfig (27) zum Anschlussstück (17) hin durch eine sich nach außen verengende Ringstruktur (28) begrenzt wird.

2. Patienteninterface nach Anspruch 1 **dadurch gekennzeichnet, dass** das Kopplungselement (1) zumindest eine Ebene (50) aufweist, die parallel zu der Anlagefläche (beispielsweise Stirn) eines Benutzers verläuft, wobei das Kopplungselement (1) sich zumindest abschnittsweise abgewinkelt von der Ebene mit einer gerundeten Kontur erstreckt und eine Aussparung (31) definiert.

3. Patienteninterface nach zumindest einem der vorhergehenden Ansprüche **dadurch gekennzeichnet, dass** die Ebenen (50, 60) parallel zueinander oder in einem Winkel zueinander verlaufen.

4. Patienteninterface nach zumindest einem der vorhergehenden Ansprüche **dadurch gekennzeichnet, dass** durch die Aufnahmemittel (5) und/oder die Aufnahmemittel (6) zumindest abschnittsweise einen gebogenen Konturverlauf aufweisen.

5. Patienteninterface nach zumindest einem der vorhergehenden Ansprüche **dadurch gekennzeichnet, dass** der gebogene Konturverlauf, von einem mittleren Bereich des Ausnahmemittels ausgehend, einen ansteigenden Krümmungsradius aufweist.

6. Patienteninterface nach zumindest einem der vorhergehenden Ansprüche **dadurch gekennzeichnet, dass** die Aufnahmemittel (5) und (6) geometrisch unterschiedlich ausgebildet sind, wobei die Aufnahmemittel als geschlossener (5) oder geschlitzter Steg (6) ausgebildet sind.

7. Patienteninterface nach zumindest einem der vorhergehenden Ansprüche **dadurch gekennzeichnet, dass** die geschlitzten Stege (6) außen und die geschlossenen Stege (5) relativ zu den geschlitzten Stegen weiter innen, näher an der Symmetrieebene S angeordnet sind, wobei die geschlitzten Stege (6) und die geschlossenen Stege (5) zumindest abschnittsweise parallel zueinander verlaufen.

8. Patienteninterface nach zumindest einem der vorhergehenden Ansprüche **dadurch gekennzeichnet, dass** die Aufnahmemittel (5), (6) unterschiedlich weit von der Stirn eines Benutzers entfernt angeordnet sind.

9. Patienteninterface nach zumindest einem der vorhergehenden Ansprüche **dadurch gekennzeichnet, dass** ein geschlitzter Steg (6) zwischen den beiden Teilstegen (6a, 6b) jeweils eine Öffnung (9) für die Bänderung bereitstellt und Öffnung so dimensioniert ist, dass die Bänderung mit ihrer flachen Seite hindurchgefädelt werden kann.

10. Patienteninterface nach zumindest einem der vorhergehenden Ansprüche **dadurch gekennzeichnet, dass** das Patienteninterface als Atemmaske ausgeführt ist, welche modular aufgebaut ist, wobei als Basis der Atemmaske (13) der Maskenkörper (14) dient, wobei dieser als Spritzgussteil aus Kunststoff relativ fest ausgeführt ist und wobei an dem Maskenkörper (14) die Stirnstütze (15), die einen sicheren Sitz der Maske auf dem Gesicht eines Patienten gewährleistet, und der Maskenwulst (16), der durch seine Passform und seine elastische Beschaffenheit für einen dichten Abschluss der Atemmaske am Patienten sorgt, ansetzen, wobei das Anschlussstück (17) die Verbindung zu einem Schlauch darstellt, über den das Atemgas von einem Beatmungsapparat zur Atemmaske transportiert wird und wobei der Schlauch mittels einer auf dem Anschlussstück (17) drehbeweglich gelagerten Hülse (18) mit dem Anschlussstück (17) verbunden ist, wobei Mithilfe von seitlich mit dem Maskenkörper verbindbaren Clips (19), die Atemmaske durch Bänder sicher am Kopf des Patienten fixiert werden kann, wobei die Atemmaske eine Symmetrie des Aufbaus entlang der vertikalen Mittelachse (s) aufweist, wobei der Maskenwulst (16) eine trianguläre Grundform aufweist.

11. Patienteninterface nach zumindest einem der vorhergehenden Ansprüche **dadurch gekennzeichnet, dass** die Stirnstütze eine an die menschliche Anatomie angepasste Formgebung aufweist, wobei das Kopfende der Stirnstütze (15) aus der, sich aus der Verbindung mit dem Maskenkörper ergebenden, Fläche in Richtung eines Patienten ragt, um auch ohne übermäßiges Neigen der gesamten Atemmaske eine ausreichende Stützfunktion an der Stirn eines Patienten zu erreichen.

12. Patienteninterface nach Anspruch 11 **dadurch gekennzeichnet, dass** der Steg (22) in seiner Breite gegenüber dem Kopfteil mit Bandankopplung (23) und dem Stirnstützenverbinder signifikant reduziert ist.

13. Patienteninterface nach zumindest einem der vorhergehenden Ansprüche **dadurch gekennzeichnet, dass** das Kopfteil der Stirnstütze in Relation zum Stirnstützenverbinder (21) zu einem Patienten hin ragt, wobei die Form des die Bandankopplung (23) beherbergenden Kopfes der Stirnstütze (2) eine Aussparung (31) aufweist, die zwischen der Stirn eines Patienten und der Bandankopplung (23) in der Stirnstütze (2) einen Raum bietet, wobei die Aussparung (31) gemeinsam mit den Strukturelementen der Bänderungsankopplung, in einer Ansicht von oben, eine Y-Form aufweist, wobei der Konturverlauf im Bereich der Aussparung (31) gerundet mit einem oder mehreren Radien verläuft, wobei entsprechend des Materials der Bänderungsankopplung und der Wandstärken die Y-Struktur eine Elastizität bieten kann, die eine Adaptation des Kopfteils der Stirnstütze (13) relativ zur Stirn des Patienten erlaubt.

## Claims

1. A patient interface with at least one coupling element (1) for a harness, wherein the coupling element (1) has at least one plane of symmetry S and at least four receiving means (5), (6) for a harness end (4) of a harness (3), wherein the receiving means (5), (6) are arranged in pairs on either side of the plane of symmetry (S) and a harness end (4) can be fastened on one side of the coupling element to the receiving means (5) or alternatively to the receiving means (6) and the receiving means (5) is arranged spatially adjacent to the receiving means (6) and closer to the plane of symmetry (S) than the receiving means (6), wherein the coupling element (1) is designed in a single piece as part of a forehead support (15) of the patient interface and the plane of symmetry (S) coincides with the longitudinal axis of the forehead support, wherein the coupling element (1) has an imagined plane (E) that is perpendicular to the plane of symmetry (S) and runs parallel to the forehead of a user, wherein a plane (50) is formed by the receiving means (5) and another plane (60) is formed by the receiving means (6), wherein the planes (50, 60) are at a distance from each other, wherein, in the region of the patient interface, a first plane (40) is defined in the region of the base of the forehead support, a second plane (50) is formed in the region of the harness coupling of the forehead support by the receiving means (5), and a third plane (60) is formed in the region of the harness coupling of the forehead support by the receiving means (6), wherein the first plane (40) is at a greater distance from the third plane (60) than from the second plane (50), wherein the planes are arranged basically parallel to each other and wherein the offset of the first plane (40) from the second plane (50) is 1 cm to 4 cm and the offset of the second plane (50) from the third plane (60) is 3 mm to 23 mm, wherein a forehead support connector (21) forms the base of the forehead support (15) and the forehead support is fastened to the mask body (13) with the forehead support connector (21),
wherein a web (22) connects the forehead support connector (21) to the coupling element (1) for a harness of the forehead support (15), and
wherein, to complete a bayonet closure located in the mask body (1), the forehead support connector (21) offers a number of closure teeth (25) corresponding in positioning and number to the channels present in the mask body,
wherein these closure teeth (25) protrude radially outward on the outer contour of a connection ring (26) and each have another radially outwardly aimed tooth, which together with a latching tooth in the mask body ensure that the mask body and forehead support are locked in the provided position,
wherein the inner contour of the forehead support connector (21) is designed as a ball cage (27) that bears the connection piece (17) in a mobile manner, wherein the ball cage (27) is delimited toward the connection piece (17) by a ring structure (28) that narrows toward the outside.

2. The patient interface according to claim 1, **characterized in that** the coupling element (1) has at least one plane (50) that runs parallel to the contact surface (for example, forehead) of a user, wherein the coupling element (1) extends at least in portions at an angle from the plane with a rounded contour and defines a recess (31).

3. The patient interface according to at least one of the preceding claims, **characterized in that** the planes (50, 60) run parallel to each other or at an angle to each other.

4. The patient interface according to at least one of the preceding claims, **characterized in that** the receiving means (5) and/or the receiving means (6) have a curved contour at least in portions.

5. The patient interface according to at least one of the preceding claims, **characterized in that** the curved contour, starting from a central region of the receiving means, has an increasing curvature radius.

6. The patient interface according to at least one of the preceding claims, **characterized in that** the receiving means (5) and (6) are designed to be geometrically different, wherein the receiving means are designed as a closed (5) or slotted web (6).

7. The patient interface according to at least one of the preceding claims, **characterized in that** the slotted webs (6) are arranged to the outside and the closed webs (5) are arranged farther to the inside relative to the slotted webs, closer to the plane of symmetry S, wherein the slotted webs (6) and the closed webs (5) run parallel to each other at least in portions.

8. The patient interface according to at least one of the preceding claims, **characterized in that** the receiving means (5), (6) are arranged at different distances from the forehead of a user.

9. The patient interface according to at least one of the preceding claims, **characterized in that**, between the two partial webs (6a, 6b), each slotted web (6) provides an opening (9) for the harness and the opening is dimensioned such that the flat side of the harness can be threaded through.

10. The patient interface according to at least one of the preceding claims, **characterized in that** the patient interface is designed as a respiratory mask, which is structured modularly, wherein the mask body (14) serves as the base of the respiratory mask (13), wherein the mask body is designed to be relatively stiff as an injection molded part made of plastic, and wherein the forehead support (15), which ensures a secure fit of the mask on the face of a patient, and the mask cushion (16), which ensures a tight termination of the respiratory mask on the patient due to its fit and its elastic quality, adjoin the mask body (14), wherein the connection piece (17) is the connection to a tube, through which the respiratory gas is transported from a ventilator assembly to the respiratory mask and wherein the tube is connected to the connection piece (17) by means of a sleeve (18) mounted in a rotationally movable manner on the connection piece (17), wherein, using clips (19) that can be connected laterally to the mask body, the respiratory mask can be fixed securely to the head of the patient by straps, wherein the respiratory mask has a symmetrical structure along the vertical center axis (s), wherein the mask cushion (16) has a triangular basic shape.

11. The patient interface according to at least one of the preceding claims, **characterized in that** the forehead support has a shape that is adapted to the human anatomy, wherein the head end of the forehead support (15) projects, in the direction of a patient, from the surface resulting from the connection to the mask body in order to achieve a sufficient supporting function on the forehead of a patient even without excessive tilting of the entire respiratory mask.

12. The patient interface according to claim 11, **characterized in that** the width of the web (22) is significantly reduced compared to the head part with the strap coupling (23) and the forehead support connector.

13. The patient interface according to at least one of the preceding claims, **characterized in that** the head part of the forehead support projects toward a patient in relation to the forehead support connector (21), wherein the shape of the head of the forehead support (2) accommodating the strap coupling (23) has a recess (31), which offers a space between the forehead of a patient and the strap coupling (23) in the forehead support (2), wherein the recess (31) together with the structural elements of the harness coupling has a Y-shape in a view from above, wherein the contour in the region of the recess (31) runs rounded with one or more radii, wherein, according to the material of the harness coupling and the wall thicknesses, the Y-structure can offer an elasticity that allows an adaptation of the head part of the forehead support (13) relative to the forehead of the patient.

## Revendications

1. Interface de patient avec au moins un élément de couplage (1) pour un agencement de bride, l'élément de couplage (1) présentant au moins un plan de symétrie S et au moins quatre moyens d'accueil (5), (6) pour une extrémité d'agencement de bride (4) d'un agencement de bride (3), les moyens d'accueil (5), (6) étant disposés en paires des deux côtés du plan de symétrie (S) et une extrémité d'agencement de bride (4) pouvant alternativement être fixée sur le moyen d'accueil (5) ou sur le moyen d'accueil (6) sur un côté de l'élément de couplage et le moyen d'accueil (5) étant disposé spatialement à proximité du moyen d'accueil (6) et plus proche du plan de symétrie (S) que le moyen d'accueil (6), l'élément de couplage (1) étant conçu d'un seule pièce comme partie d'un support de front (15) de l'interface de patient et le plan de symétrie (S) coïncidant avec l'axe longitudinal du support de front, l'élément de couplage (1) présentant, verticalement par rapport au plan de symétrie (S), un plan imaginé (E), lequel s'étend parallèlement au front d'un utilisateur, un plan (50) étant formé par les moyens d'accueil (5) et un plan (60) supplémentaire étant formé par les moyens d'accueil (6), les plans (50, 60) étant à distance l'un de l'autre, un premier plan (40) étant défini dans la zone de la base de l'appui de front, dans la zone de l'interface de patient, un deuxième plan (50) étant formé par les moyens d'accueil (5) dans la zone de couplage d'agencement de bride de l'appui de front et un troisième plan (60) étant formé par les moyens d'accueil (6) dans la zone de couplage d'agencement de bride de l'appui de front, le premier plan (40) étant à une plus grande distance du troisième plan (60) que du deuxième plan (50), les plans étant essentiellement disposés parallèlement les uns aux autres et le décalage entre le premier plan (40) et le deuxième plan (50) s'élevant à de 1 cm à 4 cm et le décalage entre le deuxième plan (50) et le troisième plan (60) s'élevant à de 3 mm à 23 mm, une attache d'appui de front (21) formant la base de l'appui de front (15) et l'appui de front étant fixé à la coque de masque (13) avec l'attache d'appui de front (21),
un montant (22) reliant l'attache d'appui de front (21) avec l'élément de couplage (1) pour un agencement de bride de l'appui de front (15) et
l'attache d'appui de front (21) offrant un certain nombre de dents de fermeture (25) correspondant en positionnement et en nombre aux canaux présents dans la coque de masque, pour compléter une fixation à baïonnette disposée dans la coque de masque (1),
ces dents de fermeture (25) se trouvant radialement à l'extérieur sur le contour extérieur d'un anneau de liaison (26) et présentant elles-mêmes respectivement une dent supplémentaire orientée radialement vers l'extérieur, laquelle assure, conjointement avec une dent d'arrêt dans la coque de masque, la fixation de la coque de masque et de l'appui de front dans la position prévue,
le contour intérieur de l'attache d'appui de front (21) étant réalisé comme une cage à billes (27), laquelle supporte de façon mobile la pièce de raccordement (17), la cage à billes (27) étant limitée, vers la pièce de raccordement (17), par une structure annulaire (28) se rétrécissant vers l'extérieur.

2. Interface de patient selon la revendication 1, **caractérisée en ce que** l'élément de couplage (1) présente au moins un plan (50), lequel s'étend parallèlement à la surface d'appui (par exemple le front) d'un utilisateur, l'élément de couplage (1) s'étendant de façon coudé avec un contour arrondi à partir du plan au moins par sections et définissant un évidement (31).

3. Interface de patient selon au moins une des revendications précédentes, **caractérisée en ce que** les plans (50, 60) s'étendent parallèlement l'un à l'autre ou avec un angle l'un par rapport à l'autre.

4. Interface de patient selon au moins une des revendications précédentes, **caractérisée en ce que** les moyens d'accueil (5) et/ou les moyens d'accueil (6) présentent un tracé de contour courbe au moins par sections.

5. Interface de patient selon au moins une des revendications précédentes, **caractérisée en ce que** le tracé de contour courbe présente un rayon de courbure croissant à partir d'une zone centrale du moyen d'accueil.

6. Interface de patient selon au moins une des revendications précédentes, **caractérisée en ce que** les moyens d'accueil (5) et (6) sont conçus géométriquement de façon différente, les moyens d'accueil étant conçus comme un montant fermé (5) ou un montant fendu (6).

7. Interface de patient selon au moins une des revendications précédentes, **caractérisée en ce que** les montants fendus (6) sont disposés à l'extérieur et les montants fermés (5) sont disposés plus à l'intérieur que les montants fendus, à une plus grande proximité du plan de symétrie S, les montants fendus (6) et les montants fermés (5) s'étendant parallèlement les uns aux autres au moins par sections.

8. Interface de patient selon au moins une des revendications précédentes, **caractérisée en ce que** les moyens d'accueil (5), (6) sont disposés à une distance différente du front d'un utilisateur.

9. Interface de patient selon au moins une des revendications précédentes, **caractérisée en ce qu'**un montant fendu (6) fournit respectivement une ouverture (9) entre les deux montants partiels (6a, 6b) pour l'agencement de bride et **en ce que** l'ouverture est dimensionnée de telle sorte que l'agencement de bride peut être enfilé par son côté plat.

10. Interface de patient selon au moins une des revendications précédentes, **caractérisée en ce que** l'interface de patient est réalisée comme un masque respiratoire, lequel est construit de façon modulaire, la coque de masque (14) servant de base pour le masque respiratoire (13), la coque de masque étant réalisée de façon relativement rigide comme pièce moulée par injection en matière plastique, et l'appui de front (15), lequel garantit une bonne stabilité du masque sur le visage d'un patient, et le bourrelet de masque (16), lequel assure une fermeture étanche du masque respiratoire sur le patient grâce à son ajustement et à sa nature élastique, étant appliqués sur la coque de masque (14), la pièce de raccordement (17) représentant le raccordement avec un tube, par lequel le gaz respiratoire est transporté d'un appareil respiratoire au masque respiratoire et le tube étant raccordé à la pièce de raccordement (17) au moyen d'une gaine (18) montée de façon mobile en rotation sur la pièce de raccordement (17), le masque respiratoire pouvant être fixé de façon sûre sur la tête du patient par des bandes, à l'aide de clips (19) pouvant être reliés latéralement à la coque de masque, le masque respiratoire présentant une symétrie de construction le long de l'axe central vertical (s), le bourrelet de masque (16) présentant une forme de base triangulaire.

11. Interface de patient selon au moins une des revendications précédentes, **caractérisée en ce que** l'appui de front présente un façonnage ajusté à l'anatomie humaine, l'extrémité de tête de l'appui de front (15) dépassant de la surface résultant de la liaison avec la coque de masque dans la direction d'un patient, afin de réaliser une fonction d'appui suffisante sur le front d'un patient sans inclinaison excessive du masque respiratoire entier.

12. Interface de patient selon la revendication 11, **caractérisée en ce que** le montant (22) est réduit de façon significative dans sa largeur par rapport à la partie de tête avec couplage de bande (23) et l'attache d'appui de front.

13. Interface de patient selon au moins une des revendications précédentes, **caractérisée en ce que** la partie de tête de l'appui de front dépasse vers un patient relativement à l'attache d'appui de front (21), la forme de la tête de l'appui de front (2) accueillant le couplage de bande (23) présentant un évidement (31), lequel offre un espace dans l'appui de front (2), entre le front d'un patient et le couplage de bande (23), l'évidement (31) présentant vu de dessus une forme en Y, conjointement avec les éléments de structure du couplage d'agencement de bride, le tracé de contour s'étendant de façon arrondi avec un ou plusieurs rayons, dans la zone de l'évidement (31), la structure en Y pouvant offrir une élasticité de façon corrélative au matériau du couplage d'agencement de bride et des épaisseurs de paroi, laquelle élasticité permet une adaptation de la partie de tête de l'appui de front (13) relativement au front du patient.
